# EUROPÄISCHE PATENTSCHRIFT

(11) **EP 1 436 025 B1**
(45) Veröffentlichungstag und Bekanntmachung des Hinweises auf die Patenterteilung: **07.03.2007**
(21) Anmeldenummer: 02785230.0
(22) Anmeldetag: 17.10.2002
(51) Int. Cl.: A61M 1/16, A61L 2/10, C02F 1/32

(54) **Vorrichtung zur Aufbereitung wässriger Flüssigkeiten**
Device for treating aqueous liquids
Dispositif de traitement de liquides aqueux

(30) Priorität: 18.10.2001 DE 10151488
(43) Veröffentlichungstag der Anmeldung: 14.07.2004
(73) Patentinhaber: Büttner, Klaus, D-25336 Klein Nordende (DE)
(72) Erfinder: Büttner, Klaus, D-25336 Klein Nordende (DE)
(74) Vertreter: Glaeser, Joachim
(86) Internationale Anmeldenummer: PCT/EP2002/011617
(87) Internationale Veröffentlichungsnummer: WO 2003/035145

(56) Entgegenhaltungen:
- WO-A-97/46271
- WO-A-99/62573
- DE-A- 19 650 118
- US-A- 2 309 124
- US-A- 3 894 236
- US-A- 4 610 782
- US-A- 5 770 147
- US-A- 6 071 473

## Beschreibung

Die Erfindung bezieht sich auf ein Vorrichtung zur Aufbereitung von wässrigen Flüssigkeiten zwecks Sterilisation und Elimination von darin enthaltenen Schadstoffen, aufweisend einen UV-Strahler und ein den UV-Strohler umgebendes Gehäuse.

Im Rahmen von humanmedizinischen Therapien, bei denen der Patient direkt oder indirekt mit Wasser oder wässrigen Lösungen in Kontakt kommt, ist eine sehr hohe Reinheit des Wassers eine absolute Notwendigkeit. Eine adäquate Wasseraufbereitung ist in der Praxis jedoch aufgrund von fehlenden technischen Realisierungsmöglichkeiten nur allzu oft nicht möglich. Dabei ist mit adäquater Wasseraufbereitung eine Eliminierung problematischer Mikroorganismen, Keime, Pilze, u.a.) und Pyrogene (Endotoxine, Exotoxine) gemeint. Zu erwähnen sind zudem Schadstoffe wie Pestizide, u.a. Dies gilt insbesondere bei Therapien, die mehrere Liter strömende wässrige Flüssigkeiten benötigen und die während der Behandlung das zuvor unzureichend aufbereitete Wasser aus dem zur Verfügung stehendem städtischen Netz beziehen. Als Beispiel sei die Hämodialyse niereninsuffizienter Patienten genannt.

Derzeit ist die Herstellung von sogenanntem "ultrareinen Wasser-Dialysat" in oben beschriebener Praxis ausschließlich unter Verwendung eines Sterilfilters in Kombination mit einem Aktivkohlefilter möglich. Dieser Aufbereitungsschritt ist jedoch sehr kostenintensiv und bietet langfristig aufgrund der Gefahr der Bildung multiresistenter Keime keine befriedigende Lösung. Obwohl die Ärzteschaft ausdrücklich ultrareines Dialysat fordert, ist dieses zum jetzigen Zeitpunkt flächendeckend nicht realisierbar.

Mit den aus der Literatur bekannten theoretischen Möglichkeiten der UV-Desinfektion ist prinzipiell eine Alternative zum Sterilfilter im Rahmen der Wasseraufbereitung für humanmedizinische therapeutische Anwendungen vorhanden. Es ist bekannt, daß die UV-Desinfektion sowohl sterilisierend als auch unter bestimmten Voraussetzungen zur Erzeugung von sogenanntem "Reinstwasser" geeignet ist. Reinstwasser wird zum Beispiel in der Halbleiterindustrie benötigt und zeichnet sich neben den Eigenschaften eines hochreinem Wassers durch einen sehr kleinen Anteil an Rohlenstoffverbindungen aus. Bei der Sterilisation durch UV-Strahlen werden lebende Mikroorganismen durch Zerstörung der DNA mit einer Wellenlänge λ=254nm abgetötet oder inaktiviert. Zudem lassen sich weitere Schadstoffe und Endotoxine durch UV-Strahlen und/oder durch Ultraschall ausgelöste und unterhaltene oxidative Prozesse in Wechselwirkung mit UV-Strahlung derart abbauen, daß sie auch für den menschlichen Organismus unschädlich sind.

Derzeit gibt es jedoch kein Gerät oder Verfahren auf der Basis der UV-Sterilsation und unter in Wechselwirkung erzeugten oxidativen Prozessen, das die Erzeugung von ultrareinem Wasser, d.h. Abtöten von Mikroorganismen, Elimination von Pyrogenen und Abbau von Schadstoffen, unter dynamischen Bedingungen, d.h. wenige Sekunden, bevor der Patient direkt oder indirekt mit dem Wasser in Kontakt kommt, ermöglicht.

Durch die vorliegende Erfindung ist erstmalig eine Vorrichtung entwickelt worden, mit dem unter dynamischen Bedingungen ultrareines Wasser hergestellt wird. Dabei ermöglicht die Erfindung, ultrareines Wasser in einem von der jeweiligen Therapieform abhängigen Zeitlimit ohne weitere Chemikalienzusätze oder kostenintensive Hilfsmittel herzustellen. Dadurch werden diese Behandlungen qualitativ auf ein deutlich höheres Niveau gehoben, was für den Patienten nicht nur die Lebensqualität steigert, sondern unter Umständen lebensrettend ist.

Dies kann beim derzeitigen Stand der Technik mit keinem Gerät oder Verfahren gelöst werden. Mit bekannten Verfahren sind lediglich einige Teilaspekte einer Wasseraufbereitung möglich:
1. Sterilisation von strömenden Flüssigkeiten durch UV-Strahlen der Wellenlänge λ=254nm.
2. Abbau von Kohlenwasserstoffen in strömenden Flüssigkeiten unter Verwendung von λ=185nm, kombiniert mit weiteren Methoden (keine Spezifizierung des Herstellers).
3. Die Sterilisation und Oxidation organischer Substanzen durch UV-Strahlen der Wellenlänge λ=254nm unter Hinzugabe von OH⁻-Radikalerzeugern wie Wasserstoffperoxid und Ozon.
4. Erzeugung von Radikalen durch Bestrahlen von Titandioxid mit λ=360nm zur Desinfektion von statischen Systemen wie Oberflächen.
5. In der Entwicklung befindliche Xenon-Lampen, die kommerziell noch nicht zu erwerben sind, zur Erzeugung von UV-Strahlen der Wellenlänge λ=170nm, die H₂O zu H und OH aufspalten. Die sterilisierende Wirkung dieser Wellenlänge ist nicht mehr gegeben.

Dokument US 6071473 beschreibt eine Vorrichtung zur Aufbereitung von wässrigen Flüssigkeiten gemäß des Oberbegriffs des Patentanspruchs.

Aufgabe der Erfindung ist die Herstellung von sterilem und endotoxinfreiem Wasser oder wässriger Lösung unter dynamischen Bedingungen mit Hilfe von UV-Strahlen, und zwar zur Verwendung in der Humanmedizin und in der Lebensmittelindustrie. Insbesondere geht es um die Herstellung eines "ultrareinen Dialysates" bei einem Durchfluss von 0 ml (statisch) bis mindestens 10.000 ml/min und vorteilhafterweise um die Herstellung von Infusionslösungen im Online-Verfahren.

Zur Lösung dieser Aufgabe dienen vorrangig folgende Merkmale:
1. Abtötung oder Inaktivieren von lebenden Mikroorganismen und Pilzen in strömendem Wasser oder wässrigen Lösungen.
2. Elimination von Pyrogenen (Endotoxine und Exotoxine) in strömendem Wasser oder wässrigen Lösungen.
3. Elimination von Pestiziden, Herbiziden und Fungiziden in strömendem Wasser oder wässrigen Lösungen.
4. Anreicherung von Dialyseflüssigkeit mit Sauerstoff zur Vitalisierung der Dialysepatienten während der Dialysebehandlung.

Um dieses Ziel zu erreichen, wird eine Vorrichtung benötigt, in der die folgenden Verfahrensabläufe stattfinden:

Die -OH-Radikal-Erzeugung wird durch die Kombination der gewählten UV-Wellenlängen und/oder einem integrierten Rührwerk, vorzugsweise einem Ultraschallgenerator, eingeleitet und aufrecht erhalten, wobei genügend -OH-Radikale erzeugt werden. Die erzeugten -OH-Radikale werden im Flüssigkeitsvolumen ausreichend gleichmäßig verteilt, um eine hohe Trefferwahrscheinlichkeit zwischen ⁻OH-Radikalen und Endotoxinen bzw. Exotoxinen zu gewährleisten, wobei dies unter statischen und dynamischen Bedingungen von wenigen ml/min. bis mindestens 10.000 ml/min. Fluss sichergestellt wird.

Dieses Ziel wird erreicht durch die Vorrichtung gemäß des Patentanspruchs.

Die Vorrichtung nach der Erfindung kann im Wesentlichen aus einem oder mehreren Hohlkörpern bestehen, durch den oder durch die zu behandelnde wässrige Lösung hindurchgeleitet wird. Er kann außerhalb eines Gerätes angeordnet oder auch in ein Gerät eingebracht werden oder aber auch integraler Bestandteil eines Gerätes sein.
Wesentlich für die Vorrichtung gemäß der Erfindung ist, dass sich in dem Hohlkörper mindestens ein UV-Strahler befindet, wobei der oder die Strahler mindestens zwei UV-Wellenlängen aus dem Spektrum 170 nm bis 260 nm in geeigneter Kombination emittieren.
Wesentlich ist ebenfalls ein chaotischer Strömungsverlauf der Flüssigkeit in dem Hohlkörper um den oder die UV-Strahler. Diese chaotische Strömung wird mit einem integrierten Ultraschallgenerator (Frequenz >18 kHz) erzeugt.
Dieser Ultraschallgenerator dient als eine Art Rührwerk. Der oder die in Kombination mit einem Ultraschallgenerator verwendeten UV-Strahler emittiert oder emittieren mindestens eine Wellenlänge aus dem Spektrum 170 bis 260 nm.
Wesentlich ist auch, dass das zu behandelnde Medium bestimmte Strömungsverläufe im Hohlkörper in Relation zu dem Strahler oder den Strahlern durchläuft, und zwar in einer relativ dünnen Schicht an der Glasoberfläche des UV-Strahlers. Die Schichtdicke der zu reinigenden wässrigen Lösung ist abhängig von den Turbulenzen, der konturierten Oberfläche des UV-Strahlers und des in der Flüssigkeit verursachten Chaos.
Wesentlich sind auch folgende Parameter, die in Relation zueinander stehen können:
Hohlkörperdurchmesser, Röhrendurchmesser des Strahlers, Kontur der Oberfläche der verwendeten Röhre, Anströmwinkel, Anzahl der Strahler, Kontur der Außenoberfläche des Strahlerschutzrohrs oder der verwendeten Strahlerschutzrohre, UV-Wellenlänge, Strahlungsleistung, Strahlungsdichte, Verweildauer des zu sterilisierenden Mediums und erzeugtes Chaos in der wässrigen Lösung im bestrahlten Raum.
Wesentlich ist weiterhin eine Einrichtung im Inneren des Hohlkörpers, die -OH-Radikale und/oder Ozon erzeugt und diese in der strömenden wässrigen Lösung sicher und gleichmäßig verteilt.
Wesentlich ist auch, dass die zu reinigende wässrige Lösung physikalisch gelösten Sauerstoff enthält oder eine Einrichtung, die Sauerstoff in das Innere bzw. in den Strömungsraum bringt, und dieser entweder aus der Umgebungsluft oder aus Sauerstoffvorratsbehältern - oder aus dem Wasser selbst gewinnt.
Der Hohlkörper hat mindestens einen Einlauf und mindestens einen Auslauf für wässrige Flüssigkeiten. Die Öffnungen können unten und/oder oben sein, sie können zentriert und/oder tangential ausgerichtet angebracht sein.

Zur Sterilisation, Endotoxinabreicherung und zur Elimination von Kohlenwasserstoffen wässriger Lösungen wird die übliche Schlauchverbindung und/oder Verrohrung aufgetrennt und die Vorrichtung gemäß der Erfindung mit geeigneten Kupplungen als Durchflusselement eingefügt.

Abhängig von dem Röhrendurchmesser, der Kontur der Innenoberfläche der verwendeten Röhre, dem Anströmwinkel, der Anzahl der Strahler, der Außenoberfläche des oder der verwendeten Strahler, der verwendeten UV-Wellenlänge, der Strahlungsleistung, der Strahlungsdichte, des chaotischen Zustandes der Flüssigkeitsmoleküle und der Verweildauer des zu des zu sterilisierenden Mediums in der Röhre, wird Sterilität bei einem Flüssigkeitsdurchsatz zwischen wenigen ml/min (statisch) und mindestens 10.000 m/min mit Hilfe der Vorrichtung gemäß der Erfindung sicher erreicht.

Grundlage der Erfindung ist die Erzeugung und sichere Verteilung von ⁻OH-Radikalen und/oder Ozon in Wasser oder wässrigen Lösungen, aus dem bzw. aus denen die Keime, Pyrogene (Endotoxine, Exotoxine) und Kohlenwasserstoffe (Düngemittel, Pestizide, Fungizide, Herbizide) entfernt werden. Dafür sind die folgenden Merkmale wesentlich:
1. Ein oder mehrere UV-Brenner, der bzw. die eine bestimmte Wellenlänge emittieren, oder die eine geeignete Kombination aus mindestens einer Wellenlänge in den Grenzwerten zwischen 170 nm und 260 nm emittieren.
2. Eine Einrichtung, die im Inneren des Hohlkörpers, in der zu behandelnden wässrigen Lösung ⁻OH-Radikale und/oder Ozon erzeugt.
3. Eine Durchströmungsführung, die sicherstellt, dass alle Flüssigkeitsbereiche mit ⁻OH-Radikalen konfrontiert werden, und/oder
4. eine geeignete Einrichtung, die die erzeugten ⁻OH-Radikale in der strömenden wässrigen Lösung sicher verteilt.

Mögliche Ausgestaltungen sind:
zu 1: Wesentlich ist die Kombination von mindestens einer UV-Wellenlänge in den Grenzwerten zwischen 170 nm und 260 nm und eine Einrichtung im Inneren des Gehäuses, die für eine turbulente Strömung und Chaos in der zu reinigenden wässrigen Lösung sorgt.
Zu 1 und 2:
   Durch die richtige Wahl der Wellenlängen, der Flüssigkeitsführung und des Sauerstoffpartialdruckes in dem Wasser oder der wässrigen Lösung wird in der zu behandelnden wässrigen Lösung nachfolgende physikalisch chemische Reaktion angestoßen und unterhalten:
   Reaktion I.:

      O₂ + hν -> O + O

      O₂ + O -> O₃
   Reaktion II.:

      O₃ + hν -> O₂ +O

      O + H₂O -> H₂O₂
   Reaktion III.:

      H₂O₂ + hν -> ⁻OH + ⁻OH
   Reaktion IV.:

      Endotoxine + ⁻OH **→H₂O + CO₂ + Abbauprodukte**
Zu 2:

   Glukoseoxidase + Glukose + Sauerstoff **→ Glukonsäure + Wasserstoffperoxyd**
Zu 2:
   Erzeugung von ⁻OH-Radikalen aus H₂O mit einer geeigneten UV-Wellenlänge

   **→ H₂O + hv H + OH**
Zu 4 und 2:
   Wasserstoffperoxyd und/oder Peroxydessigsäure wird feindosiert in den Flüssigkeitsstrom eingeleitet.
Zu 3:
   Die Absorption von mindestens einer der UV-Wellenlängen aus dem Spektrum 170 nm bis 260 nm ist in Wasser sehr hoch. Um zu gewährleisten, dass der gesamte Flüssigkeitsstrom ausreichend mit ⁻OH-Radikalen konfrontiert wird, bedarf es einer Strömungsführung, die sicherstellt, dass das zu behandelnde Wasser oder die zu behandelnde wässrige Lösung in einer solchen Schichtdicke, die die Durchdringungstiefe der verwendeten UV-Strahlen in Wasser oder der wässrigen Flüssigkeit aus dem vorgenannten Wellenbereich nicht übersteigt, und/oder einer Geometrie, die eine sichere Verteilung der gebildeten ⁻OH-Radikale gewährleistet.
   Und/oder ein Rührwerk, das ein Chaos in der wässrigen Lösung sicherstellt.
Zu 4:
   Die Absorption von mindestens einer der UV-Wellenlängen aus dem Spektrum 170 nm bis 260 nm ist in Wasser sehr hoch. Um zu gewährleisten, dass der gesamte Flüssigkeitsstrom ausreichend mit ⁻OH-Radikalen konfrontiert wird, muss gewährleistet sein, dass mindestens eine zur ⁻OH-Radikal-Erzeugung benötigte Wellenlänge aus dem Spektrum 170 nm bis 260 nm den gesamten Flüssigkeitsstrom überstreicht. Dies wird erfindungsgemäß dadurch erreicht, dass die umströmte Oberfläche des UV-Brenners oder dessen Schutzrohr in der Art von Stalaktiten oder Stalagmiten strukturiert ist. Die Oberfläche kann auch rotationssymmetrische Erhebungen aufweisen. Schließlich kann ein Rührwerk vorgesehen werden, das chaotische Verhältnisse in der wässrigen Lösung sicherstellt.
Zu 5:
   Durch geeignete Korrelationen der folgenden Merkmale: Röhrendurchmesser, Kontur der Innenoberfläche der verwendeten Röhre, Anströmwinkel, Anzahl der Strahler, Außenoberfläche des oder der verwendeten Strahler, UV-Wellenlänge, Strahlungsleistung, Strahlungsdichte, der Verweildauer des zu sterilisierenden Mediums und des in ihm erzeugten Chaos in der Röhre, wird Sterilität und Endotoxinfreiheit bei einem Flüssigkeitsdurchsatz zwischen 0 ml/min (statisch) und mindestens 10.000 ml/min sicher erreicht.

Die vorbeschriebene Vorrichtung zur Erzeugung von sterilem und endotoxinfreiem Wasser oder wässriger Lösungen eignet sich in besonderer Weise dafür, die Durchführung der therapeutischen Haemodialyse sowohl für die Patienten, das Personal und für die Umwelt sicherer zu gestalten. Das heißt dem Therapeuten wird es ermöglicht, die Therapie der Haemodialyse in den nachfolgend aufgeführten Positionen erheblich sicherer, effektiver und preiswerter zu gestalten:
1. Online-Dialyseverfahren:
   Während der Durchführung spezifischer Haemodialyse-Therapien wird dem Patienten sehr viel mehr Flüssigkeit entzogen als aus dem Flüssigkeitspotential des Patienten kontinuierlich Flüssigkeit (Substituat) wieder zugeführt werden kann. Die Online-Dialyse wurde und wird teilweise noch mit Hilfe von in Beuteln gekaufter Substituate über Bilanzierungseinrichtungen dem Patienten ausgeführt. Hierbei wird ein Teil der von dem Dialysegerät produzierten Dialyseflüssigkeit mit Hilfe von Sterilfiltern soweit gereinigt, dass sie als Substituat verwendet werden kann, was aber teuer ist. Zudem wird das Substituat aus unsteriler pyrogen- und endotoxin- / exotoxinhaltiger Dialyseflüssigkeit hergestellt. Das bedeutet, dass die Sicherheit des Patienten nur dann gewährleistet ist, wenn alle Kapillaren des verwendeten Sterilfilters ohne Mikrolecks sind.
2. Patientensicherheit:
   Durch die unsterile Dialysierflüssigkeit wird der Patient während der Dialysebehandlung erheblich belastet. Weiterhin wird bei fast allen im Markt befindlichen Dialysegeräten die Entgasung der Dialysierflüssigkeit mit dem Abwasserstrom, dem Dialysat, nach dem Venturiprinzip vollzogen. Dadurch kann es zusätzlich zur retrograden Verkeimung der Dialysierflüssigkeit kommen. Sterilfilter haben mit ca. 200h Dialysebetrieb eine relativ kurze Standzeit. D. h. spätestens alle zwei Monate muss der Sterilfilter gewechselt werden. Es gibt z. Zt. keinen Indikator, der die Erschöpfung des Filters anzeigt. Daraus ergeben sich ein Überwachungsproblem und darüber hinaus auch eine Gefahrenquelle beim Wechsel des Filters, weil die zu eliminierenden Mikroorganismen nicht getötet werden, sondern sich im Filter ansammeln. Aus diesem Grund scheint der Filter auch mit dazu beizutragen, multiresistente Keime zu erzeugen. Nicht zuletzt können Mikrolecks durch Kapillarbruch entstehen und Passagen für Mikroorganismen darstellen und somit den Patienten massiv gefährden.
3. Gefahren für das Personal:
   Zur Zeit werden, wenn überhaupt, aus Kostengründen Sterilfilter nur vor dem Dialysator eingesetzt. Das bedeutet, dass das Dialysegerät sowohl im Bereich der Dialysierflüssigkeit als auch im Abwasserbereich (Dialysat) ungeschützt ist. Das Dialysegerät bietet nun aber hervorragende Wachstumsbedingungen für eingetragene Keime. Keimansammlungen stellen für das technische Personal eine erhebliche Gefahrenquelle dar. Des weiteren kann der Filterwechsel eine Gefahrenquelle darstellen.
4. Umweltschutz und Hygiene:
   Die verbrauchte Dialysierflüssigkeit, das Dialysat, wird bislang ungereinigt in die Abwasserkanäle geleitet. Aufgrund der erheblichen Eiweißablagerungen im Abwasserrohrsystem stellt das ein grundsätzliches Problem der Dialysebetreiber dar. Diese Ablagerungen sind ein hervorragender Nährboden für alle Mikroorganismen und besonders kritisch, wenn hochinfektiöse (Hepatitis, HIV etc.) Dialysepatienten behandelt werden.

### Abhilfen gemäß der Erfindung:

Die Durchführung der Online-Dialyse wird zunächst dadurch sicherer, dass das Substituat aus bereits steriler Dialysierflüssigkeit unter nochmaliger Sterilisation und Endotoxinabreicherung hergestellt wird. Sie wird auch dadurch sicherer, dass die Mikroorganismen nicht in eine andere Matrix erhoben, sondern getötet und deren Bruchstücke im wesentlichen zu H₂O und CO₂ oxydiert werden. Es kann auch kein Mikroleck durch den Bruch von Filterkapillaren entstehen.

Es wird sowohl das Permeat, die Dialysierflüssigkeit, das Dialysat als auch die Substitutionslösung sterilisiert und von Endotoxinen gereinigt. Somit werden die folgend genannten Gefahren ausgeschlossen:
a) Belastungen des Patienten durch unsteriles und endotoxinbelastetes Dialysat.
b) Retrograde Verkeimungen bei der Entgasung der Dialysierflüssigkeit, weil die verbrauchte Dialysierflüssigkeit (Dialysat) sterilisiert wird.
c) Multiresistente Keime können sich nicht bilden, weil alle Mikroorganismen getötet und deren Bruchstücke im wesentlichen zu H₂O und CO₂ oxydiert werden.

Es werden sowohl das Permeat, die Dialysierflüssigkeit, das Dialysat als auch die Substitutionslösung sterilisiert und von Endotoxinen gereinigt, und die nachfolgend beschriebenen Gefahren ausgeschlossen und folgend genannten Vorteile erzielt werden:
a) Retrograde Verkeimungen bei der Entgasung der Dialysierflüssigkeit werden ausgeschlossen, weil die verbrauchte Dialysierflüssigkeit (Dialysat) sterilisiert wird. Somit entfallen das Kontaminationsrisiko und die Ansteckungsgefahr für den Servicetechniker.
b) Durch die wesentlich geringeren Serviceintervalle (12.000 h oder ca. 4,5 Jahre Dialysebetrieb) werden Arbeitszeit und Sekundärkontamination reduziert.
c) Die Effizienzüberwachung geschieht durch Ablesen des Indikators.
d) Durch das Töten der Mikroorganismen entfällt das Entsorgen eines mit Mikroorganismen beladenen Filters als Sondermüll.
e) Verunreinigungen durch Ablagerungen etc., die in flüssigkeitsdurchströmten Röhren grundsätzlich nicht zu vermeiden sind, treten nicht mehr auf. Durch das Ultraschallrührwerk entsteht erfindungsgemäß ein sich selbst reinigendes System.

Die Erfindung wird nachstehend anhand der Zeichnung beispielsweise erläutert:
- Fig. 1: zeigt eine Querschnittsansicht durch eine Vorrichtung gemäß der Erfindung.
- Fig. 2: zeigt eine entsprechende Querschnittsansicht einer abgewandelten Ausführungsform der Vorrichtung gemäß der Erfindung.
- Fig. 3: zeigt eine abgewandelte Ausführungsform der Vorrichtung welche nicht unter den Schutzanspruch fällt.

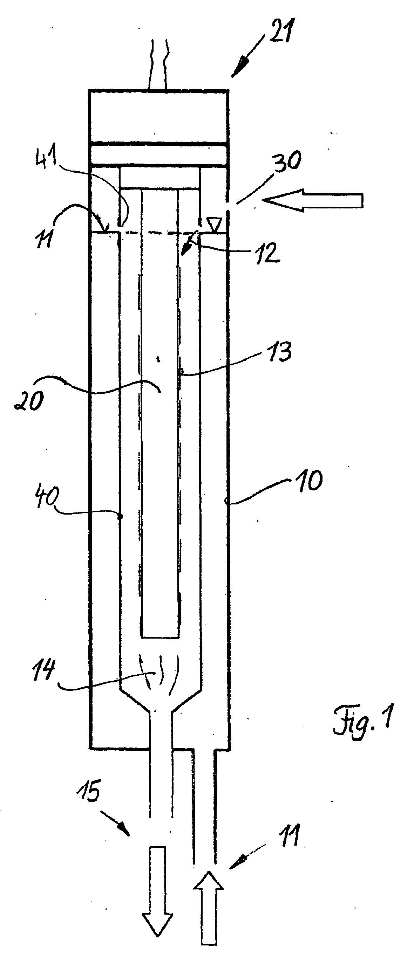

Die in den Fig. 1 bis 3 gezeigten Ausführungsformen sind im Wesentlichen rotationssymmetrisch aufgebaut, wobei die einzelnen Gehäuseteile aus Glas hergestellt worden sind.

Mit 10 ist ein Gehäuseaußenzylinder bezeichnet, in dem von unten her bei 11 die zu behandelnde Flüssigkeit eingeleitet wird. Die Einleitung kann über eine Pumpe und ein Drosselventil erfolgen, so daß ein bestimmtes Volumen pro Zeiteinheit in das Gehäuse 10 eintritt. Es stellt sich sodann im oberen Bereich des Gehäuses 10 ein Flüssigkeits-Pegel 11 ein.

Im Inneren des Zylinders 10 befindet sich ein weiterer Zylinder 40, der sich im unteren Bereich verjüngt und an einer Leitung angeschlossen ist, die abgedichtet aus dem Gehäuse 10 herausführt. Im oberen Bereich ist der Innenzylinder 40 mit Durchbrüchen 41 in gleicher Höhe versehen, wobei in der Fig. 1 zwei einander gegenüber liegende Öffnungen 41 gezeigt sind. Diese Durchbrüche oder Öffnungen können schlitzförmig sein und sorgen dafür, daß sich der Pegel 11 der sich im Zylinder 10 befindlichen wässrigen Flüssigkeit einstellt. Bei 12 ist mit dem Pfeil angedeutet, wie diese Flüssigkeit in den Innenraum des Zylinders 40 eintritt und dabei eine relativ dünne Oberflächenschicht auf dem Außengehäuse eines UV-Strahlers bildet. Die Flüssigkeit läuft an dem Gehäuse 20 nach unten und löst sich dort von dem Gehäuse bei 14 und gelangt über das bereits erwähnte Rohr bei 15 in einen Auffangbehälter.

Das Außengehäuse 10 ist bei 30 mit einem Durchgang versehen, so daß dort die Umgebungsluft in den Innenraum des Behälters 10 hineingelangen kann, so daß der Sauerstoff der Luft mit der zu behandelnden Flüssigkeit in Kontakt gelangt. In der gezeigten Ausführungsform gelangt Umgebungsluft durch die Öffnung 30 in den Behälter 10. Bei einer bevorzugten Ausführungsform kann es sich hierbei um eine Sauerstoffzufuhr handeln.

Mit 21 ist schließlich die Halterung des UV-Strahlers bezeichnet, der einerseits mit elektrischer Energie versorgt wird und andererseits auch der mechanischen Halterung der Zylinder 10 und 40 dient.

Die zu behandelnde Flüssigkeit gelangt bei 11 in das Gehäuse 10 und bildet schließlich auf der Oberfläche des UV-Strahlers 20 eine relativ dünne Schicht oder einen Film 13, der bei eingeschaltetem UV-Strahler mit der entsprechenden Strahlung beaufschlagt wird. Aufgrund der relativ geringen Dicke der Strömung kann die Strömung der Flüssigkeit gleichmäßig mit den in Frage kommenden Wellenlängen des UV-Strahlers 20 behandelt werden, so daß die gewünschte Wirkung, die Bildung der OH⁻-Radikalen mit Hilfe von physikalisch gelöstem Sauerstoff, oder wie wie bereits beschrieben, im Falle von Flüssigkeiten, die keinen physikalisch gelösten Sauerstoff beinhalten, erzielt werden kann.

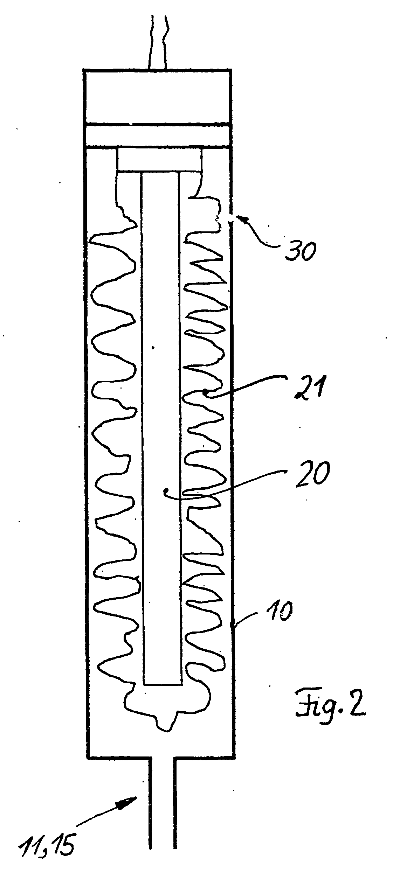

In der Fig. 2 ist eine abgewandelte Ausführungsform der Vorrichtung gemäß der Erfindung gezeigt und zwar ist dort die Außenkontur 21 des UV-Strahlers 20 so ausgebildet worden, daß die strömende Flüssigkeit einen relativ langen Weg durchlaufen muß, wobei sie auf diesem Weg intensiv bestrahlt wird, und zwar insbesondere mit der Wellenlänge 185 nm. Diese Strahlung wirkt nur in relativ kurzem Abstand von der Oberfläche des UV-Strahlers her auf die Flüssigkeit 1 ein, da bei größeren Dicken der Flüssigkeitsräume Absorptionsvorgänge stattfinden, die der OH⁻-Radikalbildung entgegenwirken. Diese Wirkung durch die vergrößerte Oberfläche des UV-Strahlers bzw. des verlängerten Weges für die Flüssigkeit kann noch dadurch erhöht werden, daß in den Strömungsweg Hindernisse eingebaut werden, die in der Flüssigkeit Turbulenzen erzeugen.

Es liegt im Rahmen der vorliegenden Erfindung, Vorrichtungen der in den Fig. 1 und 2 gezeigten Art hintereinander zu schalten, so daß in mehreren Stufen nacheinander die gewünschte Behandlung der Flüssigkeit erfolgt.

Darüber hinaus ist es auch denkbar, daß die Innenwandung des äußeren Behälters eine vom Zylinder abweichende Oberfläche erhält, etwa eine solche, die zu der Oberfläche 21 des UV-Strahlers korrespondiert, so daß die Flüssigkeit durch relativ eng bemessene Strömungsdurchgänge hindurchlaufen muß. Eine einfache Realisierung einer solchen Form wäre eine wendel- oder schraubenförmig gestaltete Oberfläche des UV-Strahlers 20 und eine entsprechend etwas größer ausgestaltete Innenoberfläche des Gehäuses 40.

Es liegt weiterhin im Rahmen der vorliegenden Erfindung, die in den Figuren 1 und 2 gezeigte Strömungsrichtung der Flüssigkeit umzukehren, so daß die Flüssigkeitssäule von unten her an dem Brennergehäuse nach oben aufsteigt.

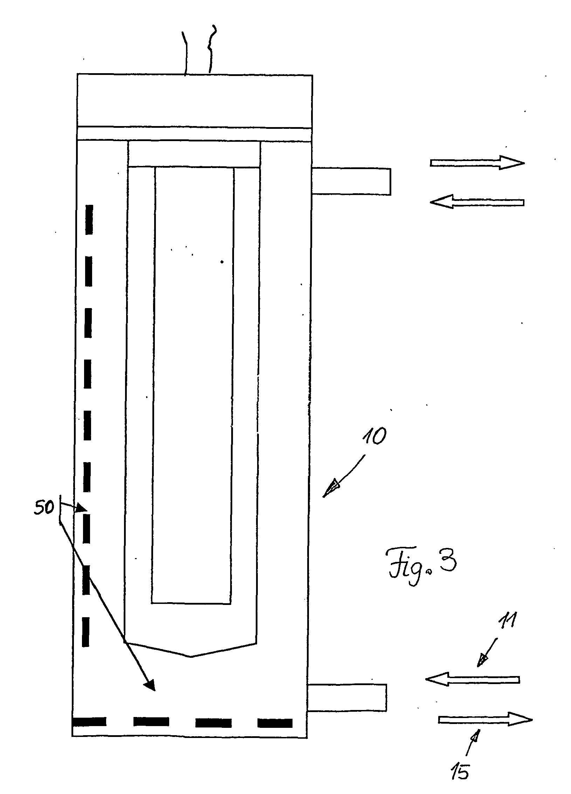

In Fig. 3 sind mit 50 mehrere Ultraschallgeber an der Seitenwand oder im unteren Bereich des Gehäuses 10 bezeichnet worden. Es könnte aber auch ein einziger parallel zum UV-Brenner 20 geführter Ultraschallstab verwendet werden.

## Patentansprüche

1. Vorrichtung zur Aufbereitung von wässrigen Flüssigkeiten zwecks Sterilisation und Elimination von darin enthaltenen Schadstoffen, aufweisend mindestens einen UV-Strahler (20) und ein den UV-Strahler umgebendes zylinderförmiges Gehäuse (10), wobei der UV-Strahler mindestens zwei Wellenlängen aus dem Spektrum 170 bis 260 nm emittieren kann, in dem zylinderförmigen Gehäuse (10) ein weiteres den UV-Strahler (20) umgebendes Innengehäuse (40) angeordnet is, wobei das Innengehäuse als Zylinder (40) ausgebildet ist, weiterhin aufweisend einen integrierten Ultraschallgenerator,
**dadurch gekennzeichnet, dass** die Frequenz des Ultraschallgenerators höher als 18 kHz ist und dass der Zylinder (40) sich einerseits im unteren Bereich verjüngt und an einer Leitung (15) angeschlossen ist, die abgedichtet aus dem Gehäuse (10) herausführt und andererseits im oberen Bereich mit schlitzförmigen, auf gleicher Höhe liegenden Durchbrüchen (41) für den Übergang der Flüssigkeit versehen ist, so dass sich der Pegel (11) der sich im Gehäuse (10) befindlichen wässrigen Flüssigkeit einstellt und dass die Flüssigkeit über die Durchbrüche (41) so auf die Oberfläche des UV-Strahlers (20) geleitet wird, dass sich dort ein dünner Fließfilm (13) bildet, wobei eine chaotische Strömung in der Flüssigkeit mittels des integrierten Ultraschallgenerators erzeugt werden kann.

## Claims

1. Device for the treatment of aqueous liquids in order to sterilise and eliminate pollutants contained therein, comprising at least one ultraviolet emitter (20) and a cylindrical housing (10) surrounding the ultraviolet emitter, said ultraviolet emitter being able to emit at least two wave lengths from the spectrum of 170 to 260 nm, a further inner housing (40) surrounding the ultraviolet emitter (20) being arranged in the cylindrical housing (10), wherein the inner housing is formed as a cylinder (40), further comprising an integral ultrasound generator, **characterised in that** the frequency of the ultrasound generator is higher than 18 kHz and that the cylinder (40) tapers in the lower area and is connected to a line (15) on the one hand, which sealed leads out of the housing (10), and is provided in the top area with slit-shaped breakthroughs (41) located at the same level for the transition of the liquid on the other hand such that the level (11) of the aqueous liquid located in the housing (10) sets, and that the liquid is guided through the breakthroughs (41) onto the surface of the ultraviolet emitter (20) such that a thin flow film (13) forms there, wherein a chaotic flow in the liquid can be generated by means of the integrated ultrasound generator.

## Revendications

1. Dispositif pour le traitement de liquides aqueux en vue de la stérilisation et de l'élimination de substances nocives y contenues, qui comprend au moins un émetteur de rayons ultraviolets (20) entouré par un boîtier cylindrique (10), étant observé que l'émetteur de rayons ultraviolets peut émettre aux moins deux longueurs d'onde différentes du spectre compris entre 170 et 260 Joules et qu'un boîtier intérieur (40) cylindrique entourant l'émetteur de rayons ultraviolets est logé dans le boîtier (10), le dispositif comprenant en outre un générateur d'ultrasons intégré, **caractérisé en ce que** la fréquence du générateurs d'ultrasons est supérieure à 18 kHz et que le cylindre (40), d'une part, diminue de manière conique dans sa partie inférieure et est raccordé à un conduit (15) sortant de manière étanche du boîtier (10), et, d'autre part, comporte dans sa partie supérieure des ouvertures (41) en forme de fentes, disposées à la même hauteur et permettant le passage du liquide, de sorte que le niveau (11) du liquide aqueux dans le boîtier (10) puisse se régler et que le liquide passe par les ouvertures (41) pour atteindre la surface de l'émetteur de rayons ultraviolets (20) et qu'il s'y forme une mince couche fluidifiée (13), un courant chaotique pouvant être produit dans le liquide au moyen du générateur d'ultrasons intégré.
